# EUROPEAN PATENT SPECIFICATION

(11) **EP 0 928 185 B1**
(45) Date of publication and mention of the grant of the patent: **03.09.2003**
(21) Application number: 97924161.9
(22) Date of filing: 03.06.1997
(51) Int. Cl.: A61K 7/48, A61K 31/20, A61K 31/23

(54) **COMPOSITIONS AND USES THEREOF**
ZUSAMMENSETZUNGEN UND DEREN VERWENDUNGEN
COMPOSITIONS ET LEURS EMPLOIS

(30) Priority: 03.06.1996 GB 9611530; 03.06.1996 GB 9611529; 03.06.1996 GB 9611531
(43) Date of publication of application: 14.07.1999
(73) Proprietor: CRODA INTERNATIONAL plc, Goole North Humberside DN14 9AA (GB)
(72) Inventor: COUPLAND, Keith, Hotham, York YO4 3UX (GB); PACKER, Claire, Elizabeth, Doncaster DN4 7EG (GB)
(74) Representative: Coates, Ian Harold
(86) International application number: GB9701496
(87) International publication number: WO97046219

(56) References cited:
- EP-A- 0 399 417
- EP-A- 0 454 102
- EP-A- 0 457 950
- WO-A-94/15464
- FR-A- 2 648 347
- GB-A- 2 118 567
- GB-A- 2 266 052
- US-A- 3 405 151
- US-A- 5 178 873

## Description

This invention relates to certain new compositions, some of which are pharmaceutical compositions. More particularly, the invention relates to compositions containing stearidonic add or derivatives thereof. The invention also relates to the use of such compositions.

Ultraviolet (UV) light in the wavelength range of 290 nm to 340 nm is covered by the ranges UVB (290 nm to 320 nm) and UVA2 (320 nm to 340 nm); UV light in this range is erythemognic. Exposure to small amount of UVB can be beneficial, since it is required for synthesis of vitamin D in the skin. However, larger amounts of UV radiation cause sunburn, which is characterised by erythema, pain, swelling and blistering; in extreme cases there is epidermal necrosis.

Sunburn is a classical cutaneous inflammation whose pathogenesis is not completely understood. The release of inflammatory mediators including eicosanoids and cytokines seems to be important UVB exposure can also compromise the immune system. Chronic UV exposure results in accelerated skin aging called photoaging; it can also lead to the development of skin cancer. The major target in UV induced carcinogenesis is probably DNA. However, the suppressed immune system could play an important role in the pathogenesis of these neoplasms. In addition to necrosis induced by UV exposure, there is evidence that even acute exposure can result in apoptosis (programmed cell death) of affected keratinocvtes.

Cutaneous inflammation caused by UVB is mediated by various cytokines such as tumour necrosis factor-alpha (TNFα) and interlukin 1α (IL1α). In addition, UVB induces the release of arachidonic acid from cell membrane phospholipids, which is oxidised via the lipoxygenase and cyclooxygenase pathways to inflammatory metabolites; examples of these include leukotriene B₄ (LTB₄) and prostaglandin E₂ (PGE₂). Since human keratinocytes lack the components to produce LTB₄, PGE₂ is probably the more important inflammatory metabolite in epidermis.

US patent specification number 5 178 873 discloses the inhibition by stearidonic acid of phospholipase A₂, and the consequential use of stearidonic acid as an anti-inflammatory agent. International patent specification number WO 94/15464 relates to structured lipids comprising a combination of fatty acids, one of which is stearidonic acid. French patent specification number 2 648 347 discloses a tripartite composition for treating sunburn, one component of which is a polyunsaturated fatty acid, an example of which is stearidonic acid, and the other two components comprise another type of vegetable oil and vitamin E. European patent specification number 399 417 relates to the preparation of at least 90% pure stearidonic acid by using urea and chromatagraphing. European patent specification number 454 102 relates to the treatment of schizophrenia or tardive dyskinesia with, for example, a GLA and stearidonic acid combination. Finally, European patent specification number 457 950 relates to stearidonic acid and its derivatives useful in inflammatory diseases.

We have now found that, unexpectedly, stearidonic acid (and derivatives thereof) is useful in inhibiting the cyclo-oxygenase pathway. Accordingly, the present invention provides the use of stearidonic acid, or a physiologically acceptable derivative thereof, in the manufacture of a medicament for inhibiting the cyclo-oxygenase pathway. This enables stearidonic acid (and derivatives thereof) to be used for treating skin inflammation, particularly skin inflammation caused by radiation, such as UV radiation. Stearidonic acid is particularly useful in treating burns, particularly first degree burns such as sunburn; it can also be used prophylactically as part of a sunscreen compound. The present invention therefore further provides the use of a composition as defined hereinbelow in the manufacture of a medicament for the treatment or prevention of sunburn.

Stearidonic acid (SA) is a polyunsaturated fatty acid of the n3 family; it is an essential fatty acid. Chemically, it can be described as 6c9c12c15c-octadecatetraenoic acid or 18:4n3. Stearidonic acid can be formed in the human body by the desaturation of α-linolenic acid (18:3n3) with the enzyme Δ6 desaturase. There are occasions when the activity of this enzyme in the body may be too low to produce sufficient stearidonic acid. In these circumstances, compositions containing stearidonic acid may be taken as a dietary supplement. Stearidonic acid is known to inhibit the 5-lipoxygenase pathway and to moderate production of LTB₄ from leukocytes. However, it was not previously known that stearidonic acid would inhibit the cyclo-oxygenase pathway.

According to one aspect of the invention, there is provided a composition comprising an oil extracted from the seeds of the genus *Echium,* which oil is capable of greater inhibition of UVB-induced PGE₂ release than borage seed oil and which composition comprises in the range of from about 0.1 to 20% stearidonic acid triglycerides by weight of the composition, which stearidonic acid triglycerides may optionally have been converted to a derivative thereof and, optionally, a physiologically acceptable carrier therefor. In particular, there is provided a sunscreen composition comprising stearidonic acid, or a physiologically acceptable derivative thereof, in combination with a UV blocking and/or UV absorbing material.

It is particularly preferred that the sunscreen composition includes one or more of: a moisturiser, an emollient, an emulsifier, a preservative, a dispersant, a viscosity modifier, a herbal extract, a solvent, a chelating agent, an antioxidant, a water-proofing agent, a pH adjuster, a perfume, and a protein.

It is particularly preferred that the sunscreen composition includes one or more of: titanium dioxide; zinc oxide; benzophenone-3; benzophenone-4; octyl methoxycinnamate (Parsol 1789); 3,3,5-trimethylcyclohexyl salicylate; carbomer; hydroxyethyl cellulose; lanolin alcohols; cetyl phosphate; fatty alcohols; C₁₂ to C₁₅ alkyl benzoate; cyclomethicone; caprylic/capric triglycerides; mineral oil; glycerin; vitamin E; and isopropyl mysristate.

The precise formulation of the sunscreen composition depends on the form required for the composition. Broadly, it may be in the form of a solid or a liquid suitable for topical application. Typically, the composition is provided in the form of a cream, an emulsion or a gel; however, the composition may be provided in other forms, such as a solid stick.

According to another aspect of the invention, we provide a pharmaceutical composition for treating skin inflammation caused by burns by exposure to sunlight or by exposure to UV radiation, comprising stearidonic acid, or a physiologically acceptable derivative thereof, and a physiologically acceptable carrier.

The carrier will include materials normally present in formulations for treating bums, such as an antiseptic compounds, emollients, inorganics, humectants, moisturisers, anti-inflammatory agents, vitamins, preservatives, pH adjusters, proteins, herbal extracts, carriers/solvents, soothing/cooling agents, antioxidants, perfumes, emulsifiers and viscosity modifiers. Specific examples of useful materials include glycerine, triethanolamine stearate, vitamin E, lanolin, zinc oxide, allantoin, calamine, sodium lactate, water, lactic acid, pro-vitamin B5 and menthol.

The formulation of the carrier also depends on the form required for the pharmaceutical composition. Broadly, the pharmaceutical composition may be in the form of a solid or a liquid suitable for topical application. Typically, the pharmaceutical composition is provided in the form of a cream, an emulsion or a gel; however, the pharmaceutical composition may be provided in other forms, such as a solid stick.

The amount of the stearidonic acid (or derivative) in the sunscreen composition or the pharmaceutical composition depends upon the way the composition is to be used. However, the compositions according to the invention typically contain 0.1 to 20 wt% of the stearidonic acid, or derivative; preferably 0.2 to 10 wt%.

The compositions according to the invention may be provided in a bottle, a tube or any other suitable packaging. The container for the compositions may be provided with dispensing means for dispensing the composition. Any known form of dispensing means may be used. When the composition is a liquid, it may be desirable to employ a dispensing means that can dispense it in the form of a spray.

Whilst any physiologically acceptable derivative of stearidonic acid may be used in the present invention, it is preferred that the derivative is an ester of stearidonic acid. More preferably, the ester comprises an ester of stearidonic acid and an alcohol, particularly an ester of stearidonic acid and a polyol.

It is particularly preferred that the derivative is the triglyceride of stearidonic acid. This triglyceride may be obtained from a number of sources. In one embodiment, it can be extracted from the seeds of the Boraginaceae family, especially the genus "Echium". These seeds have been found to contain a rich source of stearidonic acid. This is described in greater detail in our copending PCT patent application of even date entitled "Vegetable Oil Composition".

The compositions according to the invention may be incorporated in a wide variety of personal care or health care formulations.

According to another aspect of the invention, there is provided the use of stearidonic acid, or a physiologically acceptable derivative thereof, in a sunscreen composition.

According to another aspect of the invention, there is provided the use of stearidonic acid, or a physiologically acceptable derivative thereof, for the manufacture of a medicament for treating skin inflammation cause by burns.

According to another aspect of the invention, there is provided the use of stearidonic acid, or a physiologically acceptable derivative thereof, for the manufacture of a medicament for treating skin inflammation caused by exposure to UV radiation, or by exposure to sunlight.

Such medicaments may be in a form for topically applying stearidonic acid, or a physiologically acceptable derivative thereof, to an inflamed area.

According to another aspect of the invention, there is provided the use of stearidonic acid, or a physiologically acceptable derivative thereof, for the manufacture of a medicament for inhibiting the cyclo-oxygenase pathway.

Broadly, the invention may be applied to treat skin irritation caused by the over-exposure to a wide variety of radiation, including the UV radiation, infra-red radiation, and radiation used during chemotherapy.

The term "sunscreen composition" as used in this specification includes materials which substantially completely block UV radiation and includes materials which only partially block UV radiation.

### Example 1

This example illustrates one way to make the triglyceride derivative of stearidonic acid. 10 kg of the seeds of Echium plantagineum were crushed and the oil was extracted with 15 litres of petroleum ether (BP 40-60°C). The petroleum ether extract was evaporated to yield 1741 g of a golden yellow oil. The oil was converted to the corresponding fatty acid methyl esters and was analysed by gas chromatography. The lipid profile was as follows:

| Fatty Acid | Fatty Acid Content |
|---|---|
| 16:0 | 7.2% |
| 18:0 | 4.0% |
| 18:1 | 18.2% |
| 18:2 (LA) | 16.5% |
| 18:3 (GLA) | 11.8% |
| 18:3 (ALA) | 28.9% |
| 18:4 (SA) | 12.2% |
| Other | 1.2% |

### Example 2

Synthetic human skin grown from human fibroplasts has been demonstrated to be useful as a model for assessing phototoxity. This model was used to evaluate the inhibiting action of topically applied lipids.

Duplicate 9 x 9 mm Model ZK1301 tissues (obtainable from Advanced Tissue Sciences, La Jolla, California, USA) were dosed with 3 microlitres of test oil. Control tissues were untreated. The test oils were: blackcurrant seed oil; borage seed oil (this is a Boraginaceae oil with no stearidonic acid); marine oil; the oil from Example 1; and the oil from Example 2. The tissues were incubated for 24 hours at 37°C in an atmosphere with 5% CO₂ and greater than 90% humidity. Half the tissues were then exposed to 4 J/cm²UVB radiation using a Dr Honle Solar simulator to stimulate the inflammatory response.

The tissues were then incubated for a further 24 hours. The tissue supematants were collected and the tissues were evaluated for cell viability by an MTT (3(4,5-dimethyl thiazol-2-yl) 2,5-diphenyl tetrazolin bromide) dye reduction assay, using the Skin MTT Assay Kit ZA0022 (available from Advanced Tissue Sciences, La Jolla, California, USA). This protocol is based on a published method (T.Mosmann, J.Immunol. Meth. 65 55 (1983)).

A PGE₂ assay was performed in the supernatants. PGE₂ is a strongly pro-inflammatory eicosanoid; its release is indicative of membrane perturbation events that activate phospholipase A₂ to release arachidonic acid. Arachidonic acid is the precursor to PGE₂. The PGE₂ assay on the supernatant was carried out using a PGE₂ assay kit ZA0050, method number 6-FRD0019/Rev 002 (available from Advanced Tissue Sciences, La Jolla, California, USA). This method has been described by E. Granstrom *et al* in Prostaglandin Thrombox. Res. 5 119 (1978).

The results of the MTT Assay were as follows:

| Test Material | Mean MTT Optical Density | |
|---|---|---|
| | No UVB | UVB |
| Control | 1.52 | 1.25 |
| Blackcurrant seed oil | 2.54 | 1.30 |
| Borage seed oil | 1.63 | 1.39 |
| Marine Oil | 1.45 | 1.27 |
| Oil from Example 1 | 1.47 | 1.36 |
| Oil from Example 2 | 1.53 | 1.45 |

None of these materials produced any notable cytotoxicity or phototoxicity.

The results of the PGE₂ assay were as follows:

| Test Material | Mean PGE₂ Release (pg/ml) | |
|---|---|---|
| | No UVB | UVB |
| Control | 1300 | 17000 |
| Blackcurrant seed oil | 5100 | 15000 |
| Borage seed oil | 1400 | 16000 |
| Marine Oil | 3100 | 19000 |
| Oil from Example 1 | 2400 | 8100 |
| Oil from Example 2 | 2100 | 9600 |

These results demonstrate that UVB irradiation of control tissue caused a 13-fold increase in release of PGE_{2.} Pre-incubation with the oil from Example 1 blocked this by 50%. It will be seen that the oils from Example 1 are much more effective than blackcurrant seed oil. The main difference in the blackcurrant seed oil is that it has much less stearidonic acid than the oils in Example 1 (typically blackcurrant seed oil contains 3 to 4 wt% of stearidonic acid).

### Example 3

A sunscreen oil was prepared from the following ingredients:

| | wt% |
|---|---|
| Butyl methoxydibenzoyl methane (Parsol 1789)(1) | 2.0 |
| Octyl methoxycinnamate (Parsol MCX)(1) | 7.5 |
| Benzophenone-3 (Uvinul M40)(2) | 4.5 |
| PPG-2 myristyl ether propionate (Promyristyl PM3)(3) | 10.0 |
| Oil from Example 1 | 2.0-10.0 |
| Caprylic/capric trigylcerides (Crodamol GTCC) | to 100 |
| Perfume, preservatives, colour | qs |

| | |
|---|---|
| (1) From Givaudan | |
| (2) From BASF | |
| (3) From Croda | |

The sunscreen oil was formed by blending the ingredients, whilst heating gently. The resultant blend was stirred to cool.

### Example 4

A physical block sunscreen cream was prepared from the following ingredients:

| Oil Phase: | wt% |
|---|---|
| Myristyl myristate (Crodamol MM)(1) | 1.5 |
| Dimethicone (silicone 200/200)(2) | 7.0 |
| Glyceryl stearate (GMS N/E DP2186)(1) | 2.5 |
| Stearic acid (Crosterene SA4130)(1) | 3.0 |
| PVP/eicosene copolymer (Antaron V220)(3) | 0.5 |
| Non-ionic emulsifying blend (Polawax GP200 DP2307)(1) | 2.0 |
| Octyl hydroxy stearate (Crodamol OHS)(1) | 5.0 |
| Oil from Example 1 | 2.0-10.0 |
| Super-refined shea butter(1) | 5.0 wt% |
| Caprylic/capric trigylcerides (Crodamol GTCC)(1) | 9.0 wt% |
| Titanium dioxide | 5.0 wt% |

| Water Phase: | |
|---|---|
| Deionised water | to 100 wt% |
| Flobeads (CL-2080)(5) | 5.0 |
| Glycerine | 3.0 |
| Triethanolamine | 0.9 |
| Perfume, preservatives, colour | qs |

| | |
|---|---|
| (1) From Croda | |
| (2) From Dow Corning | |
| (3) From GAF (4) From K&K Greef | |
| (5) From Landsdowne Chemicals | |

The sunscreen cream was made by heating the oil phase (including titanium dioxide) and water phase separately to 80-85°C, then adding the water phase to the oil phase with vigorous agitation. The triethanolamine was then added. At 45°C the Flobeads were added. The stirring was continued until the temperature reached about 55°C, and then the composition was passed through suitable homogenising equipment, such as a triple roll mill.

### Example 5

An antioxidant cream was prepared from the following ingredients:

| Oil Phase: | wt% |
|---|---|
| Cosmowax D (cetearyl alcohol (and) Cetearyth 20)(1) | 2.10 |
| Crillet 3 (Polysorbate 60)(1) | 2.50 |
| Crodacol C90 (cetyl alcohol)(1) | 2.50 |
| Crodamol GTCC (capric/caprylic trigylcerides)(1) | 7.50 |
| Vitamin E(2) | 0.50 |
| Ascorbyl palmitate (2) | 0.20 |
| SR Echium Oil (1) | 2.00 |
| Water Phase: | |
| Cellosize QP30000H (hydroxyethyl cellulose)(3) | 0.50 |
| Croderol GA7000 (Glycerin)(1) | 2.00 |
| Deionised water | to 100 wt% |
| Perfume, Preservative, colour | qs |

| | |
|---|---|
| (1) From Croda | |
| (2) From Roche | |
| (3) From Union Carbide | |

The oil and water phases were heated separately to 65-70°C, then the water phase was added to the oil phase while stirring. The composition was then stirred to cool.

### Example 6

A cooling aftersun cream was prepared from the following ingredients:

| Oil Phase: | wt% |
|---|---|
| Crodamol IPM (isopropyl stearate)(1) | 5.00 |
| Crodamol CAP (cetearyl octanoate)(1) | 2.00 |
| SR Trichodesma Oil(1) | 2.00 |
| Polawax GP200 (non-ionic emulsifying wax)(1) | 2.00 |
| Cithrol GMS A/S (glyceryl stearate (and) PEG-100 stearate)(1) | 3.00 |
| Silicone Fluid 200/100 cs (dimethicone)(2) | 0.50 |
| Menthol | 0.10 |
| Allantoin | 0.25 |

| Water Phase: | |
|---|---|
| Carbopol 934 (Carbomer 934)(3) | 0.25 |
| Ethanol DEB 100 | 2.00 |
| Triethanolamine 99% | to pH 6.5 |
| Deionised water | to 100% |
| Perfume, preservative, colour | qs |

| | |
|---|---|
| (1) From Croda | |
| (2) From Dow Corning | |
| (3) From B F Goodrich | |

The carbopol should first be hydrated in water at about 60-65°C, then the remaining water-phase ingredients should be added. Both phases should then heated to 65-70°C, and then the water phase should be added to the oil phase whilst stirring. Finally, the composition should be neutralised with the amine and stirred to cool.

### Example 7

A calamine cream was prepared from the following ingredients:

| Oil Phase: | wt% |
|---|---|
| Polawax GP200 (non-ionic emulsifying wax)(1) | 8.00 |
| Crillet 3 (Polysorbate 60)(1) | 1.00 |
| SR Trichodesma oil(1) | 2.00 |
| Crodamol GTCC (capric/caprylic triglycerides)(1) | 3.00 |

| Water Phase: | wt% |
|---|---|
| Distilled Witch Hazel | 10.00 |
| Croderol GA7000(1) | 5.00 |
| Zinc oxide | 2.00 |
| Calamine | 2.00 |
| Deionised water | to 100 |
| Perfume, preservative, colour | qs |

The oil and water phases were heated separately to 65-70°C. The two phases are then combined while stirring. The zinc oxide and calamine are then added under high shear. Finally, the composition was stirred to cool.

Whilst certain embodiments of the invention have been described above, it will be appreciated that modifications can be made.

## Claims

1. A composition comprising an oil extracted from the seeds of the genus *Echium*, which oil is capable of greater inhibition of UVB-induced PGE₂ release than borage seed oil and which composition comprises in the range of from about 0.1 to 20% stearidonic acid triglycerides by weight of the composition, which stearidonic acid triglycerides may optionally have been converted to a derivative thereof; and, optionally, a physiologically acceptable carrier therefor.

2. A composition according to claim 1, wherein the oil is an extract from seeds of *Echium plantagineum.*

3. A composition according to claim 2, which oil is capable of inhibiting about 50% of UVB-induced PGE₂ release.

4. A composition according to any preceding claim, wherein the derivative of stearidonic acid is an ester.

5. A composition according to any preceding claim, wherein the derivative of stearidonic acid is an ester thereof with a polyol.

6. A composition according to any preceding claim further comprising a UV blocking and/or UV absorbing material.

7. A composition according to any preceding claim, comprising in the range of from 0.1 to 20 wt% of the stearidonic acid or derivative thereof.

8. A composition according to any preceding claim, comprising in the range of from 0.2 to 10 wt% of the stearidonic acid or derivative thereof.

9. A composition according to any preceding claim, in a form suitable for personal care or health care use.

10. A composition according to any preceding claim, in a form suitable for topical use.

11. A composition according to any preceding claim, in a form selected from creams, emulsions, gels and solid sticks.

12. A sunscreen composition according to any of claims 1 to 11, comprising one or more of: titanium dioxide; zinc oxide; benzophenone-3; benzophenone-4; octyl methoxycinnamate (Parsol 1789); 3,3,5-trimethylcyclohexyl salicylate; carbomer; hydroxethyl cellulose; lanolin alcohols; cetyl phosphate; fatty alcohols; C₁₂ to C₁₃ alkyl benzoate; cyclomethicone; caprylic/capric triglycerides; mineral oil; glycerin; vitamin E; isopropyl myristate; a chelating agent; a water-proofing agent; and a perfume

13. A pharmaceutical composition according to any of claims 1 to 11, comprising one or more of: an antiseptic compound; an inorganic; a humectant; an anti-inflammatory agent; vitamin; triethanolamine stearate; lanolin; allantoin; calamine; sodium lactate; water; lactic acid; pro-vitamin B5; and menthol.

14. A composition according to any of claims 1 to 13, comprising one or more of: a moisturiser, an emollient, an emulsifier, a preservative, a dispersant, a viscosity modifier, a herbal extract, a solvent, an antioxidant, a pH adjuster and a protein.

15. The use of stearidonic acid, or a physiologically acceptable derivative thereof, in the manufacture of a medicament for inhibiting the cyclo-oxygenase pathway.

16. The use according to claim 15, wherein the stearidonic acid or derivative thereof is from seeds of the genus *Echium*.

17. The use of a composition according to any of claims 1 to 14 in the manufacture of a medicament for the treatment or prevention of sunburn.

18. The use of a composition according to any of claims 1 to 14 in the manufacture of a medicament for the treatment or prevention of skin inflammation caused by exposure to UV radiation.

19. The use of a composition according to any of claims 1 to 14 in the manufacture of a medicament for inhibiting the cyclo-oxygenase pathway

## Patentansprüche

1. Zusammensetzung, die ein Öl enthält, welches aus den Samen der Gattung *Echium* extrahiert worden ist, wobei das Öl in der Lage ist eine größere Hemmung gegenüber einer UVB induzierten PGE₂ Freigabe zu ergeben als Borretschsamenöl und wobei die Zusammensetzung zum Inhalt hat; Triglyceride der Stearidonsäure in einem Mengenbereich, der von etwa 0,1 bis 20% des Gewichtes der Zusammensetzung reicht, und die Triglyceride der Stearidonsäure wahlweise in ein Derivat derselben umgewandelt worden sein können; und, wahlweise, einen physiologisch annehmbaren Träger für dieselbe.

2. Zusammensetzung gemäß Anspruch 1, bei welcher das Öl ein Extrakt aus den Samen von *Echium plantagineum* darstellt.

3. Zusammensetzung gemäß Anspruch 2, bei welcher das Öl in der Lage ist ungefähr 50% der von einer UVB induzierten PGE₂ Freigabe zu hemmen.

4. Zusammensetzung gemäß irgendeinem der vorhergehenden Ansprüche, bei welcher das Derivat der Stearidonsäure ein Ester ist.

5. Zusammensetzung gemäß irgendeinem der vorhergehenden Ansprüche, bei welcher das Derivat der Stearidonsäure ein Ester derselben mit einem Polyol ist.

6. Zusammensetzung gemäß irgendeinem der vorhergehenden Ansprüche, welche ferner ein UV blockierendes und / oder ein UV absorbierendes Material enthält.

7. Zusammensetzung gemäß irgendeinem der vorhergehenden Ansprüche, welche eine Menge in dem Bereich von 0,1 bis 20 Gew.-% der Stearidonsäure oder eines Derivats derselben enthält.

8. Zusammensetzung gemäß irgendeinem der vorhergehenden Ansprüche, welche eine Menge in dem Bereich von 0,2 bis 10 Gew.-% der Stearidonsäure oder eines Derivats derselben enthält.

9. Zusammensetzung gemäß irgendeinem der vorhergehenden Ansprüche, welche in einer Form vorliegt, die geeignet ist für den Gebrauch bei der personellen Pflege oder bei der Gesundheitspflege.

10. Zusammensetzung gemäß irgendeinem der vorhergehenden Ansprüche, welche in einer Form vorliegt, die geeignet ist für den topischen Gebrauch.

11. Zusammensetzung gemäß irgendeinem der vorhergehenden Ansprüche, welche in einer Form vorliegt, die ausgewählt worden ist unter Creme, Emulsion, Gel und festem Stift.

12. Zusammensetzung einer Sonnencreme gemäß irgendeinem der Ansprüche 1 bis 11, welche eine oder mehrere der folgenden Komponenten enthält: Titandioxyd; Zinkoxyd; Benzophenon-3; Benzophenon-4; Octylmethoxycinnamat (Parsol 1789); 3,3,5-Trimetbylcyclohexylsalicylat; Carbomer; Hydroxyethylzellulose; Lanolinalkohole, Cetylphosphat; Fettalkohole; C₁₂ bis C₁₃ Alkylbenzoate; Cyclomethicon; Capryl-/Caprintriglyceride; Mineralöle; Glycerin; Vitamin E; Isopropylmyristat; Chelatbildner; Hydrophobiermittel; und ein Parfüm.

13. Pharmazeutische Zusammensetzung gemäß irgendeinem der Ansprüche 1 bis 11, welche eine oder mehrere der folgenden Komponenten enthält: antiseptische Verbindung; anorganisches Mittel; Befeuchtungsmittel, entzündungshemmendes Mittel; Vitamin; Triethanolaminstearat; Lanolin; Allantoin; Calamin; Natriumlaktat; Wasser; Milchsäure; Provitamin B₅: und Menthol.

14. Zusammensetzung gemäß irgendeinem der Ansprüche 1 bis 13, welche eine oder mehrere der folgenden Komponenten enthält: Befeuchtungsmittel; Weichmacher; Emulgiermittel; Konservierungsmittel; Dispergiermittel; Viskositätsveränderer; Pflanzenextrakt; Lösungsmittel; Antioxydationsmittel; pH Regulierer und Protein.

15. Die Benutzung der Stearidonsäure oder eines physiologisch annehmbaren Derivats derselben bei der Herstellung eines Medikaments zur Hemmung der Cyclooxygenasebahn.

16. Die Benutzung gemäß Anspruch 15, bei welcher die Stearidonsäure oder das Derivat derselben aus den Samen der Gattung *Echium* stammt.

17. Die Benutzung der Zusammensetzung gemäß irgendeinem der Ansprüche 1 bis 14 bei der Herstellung eines Medikaments für die Behandlung oder die Vorbeugung von Sonnenbrand.

18. Die Benutzung der Zusammensetzung gemäß irgendeinem der Ansprüche 1 bis 14 bei der Herstellung eines Medikaments für die Behandlung oder die Vorbeugung von Hautentzündungen, die durch ein Belichten mit UV Strahlen verursacht werden.

19. Die Benutzung der Zusammensetzung gemäß irgendeinem der Ansprüche 1 bis 14 bei der Herstellung eines Medikaments zur Hemmung der Cyclooxygenasebahn.

## Revendications

1. Composition comprenant une huile extraite de graines du genre *Echium*, laquelle huile est apte à une inhibition de la libération de PGE₂ induite par UVB supérieure à celle d'une huile de graines de bourrache et laquelle composition comprend dans l'intervalle d'environ 0,1 à 20% de triglycérides d'acide stéaridonique en poids de la composition, lesquels triglycérides d'acide stéaridonique peuvent éventuellement avoir été convertis en un dérivé de celui-ci; et, facultativement, un excipient physiologiquement acceptable pour celle-ci.

2. Composition suivant la revendication 1, dans laquelle l'huile est un extrait de graines de *Echium plantagineum*.

3. Composition suivant la revendication 2, dans laquelle l'huile est apte à une inhibition d'environ 50% de la libération de PGE₂ induite par UVB.

4. Composition suivant l'une quelconque des revendications précédentes, dans laquelle le dérivé de l'acide stéaridonique est un ester.

5. Composition suivant l'une quelconque des revendications précédentes, dans laquelle le dérivé de l'acide stéaridonique est un ester de celui-ci avec un polyol.

6. Composition suivant l'une quelconque des revendications précédentes, comprenant en outre un matériau bloquant les UV et/ou absorbant les UV.

7. Composition suivant l'une quelconque des revendications précédentes, comprenant dans l'intervalle de 0,1 à 20% en poids de l'acide stéaridonique ou d'un dérivé de celui-ci.

8. Composition suivant l'une quelconque des revendications précédentes, comprenant dans l'intervalle de 0,2 à 10% en poids de l'acide stéaridonique ou d'un dérivé de celui-ci.

9. Composition suivant l'une quelconque des revendications précédentes, sous une forme appropriée pour un usage de soins personnels ou de soins de santé.

10. Composition suivant l'une quelconque des revendications précédentes, sous une forme appropriée pour un usage topique.

11. Composition suivant l'une quelconque des revendications précédentes, sous une forme choisie parmi des crèmes, des émulsions, des gels et des sticks solides.

12. Composition de crème solaire suivant l'une quelconque des revendications 1 à 11, comprenant un ou plusieurs éléments parmi: le dioxyde de titane; l'oxyde de zinc; la benzophénone-3; la benzophénone-4; le méthoxycinnamate d'octyle (Parsol 1789); le salicylate de 3,3,5-triméthylcyclohexyle; un carbomère; une hydroxyéthylcellulose; des alcools de lanoline; le phosphate de cétyle; des alcools gras; un benzoate d'alkyle C₁₂ à C₁₃; la cyclométhicone; des triglycérides capryliques/capriques; une huile minérale; la glycérine; la vitamine E; le myristate d'isopropyle; un agent chélatant; un agent étanche à l'eau; et un parfum.

13. Composition pharmaceutique suivant l'une quelconque des revendications 1 à 11, comprenant un ou plusieurs éléments parmi: un composé antiseptique; un composé inorganique; un agent humectant; un agent anti-inflammatoire; une vitamine; le stéarate de triéthanolamine; la lanoline; l'allantoïne; la calamine; le lactate de sodium; l'eau; l'acide lactique; la pro-vitamine B5; et le menthol.

14. Composition suivant l'une quelconque des revendications 1 à 13, comprenant un ou plusieurs éléments parmi: un hydratant; un émollient; un émulsifiant; un conservateur; un dispersant; un agent modifiant la viscosité; un extrait d'herbes; un solvant; un antioxydant; un agent d'ajustement du pH; et une protéine.

15. Utilisation d'acide stéaridonique, ou d'un dérivé physiologiquement acceptable de celui-ci, dans la fabrication d'un médicament pour inhiber la voie de cyclo-oxygénase.

16. Utilisation suivant la revendication 15, dans laquelle l'acide stéaridonique ou un dérivé de celui-ci est issu de graines du genre *Echium*.

17. Utilisation d'une composition suivant l'une quelconque des revendications 1 à 14, dans la fabrication d'un médicament pour le traitement ou la prévention d'un coup de soleil.

18. Utilisation d'une composition suivant l'une quelconque des revendications 1 à 14, dans la fabrication d'un médicament pour le traitement ou la prévention d'une inflammation de la peau entraînée par une exposition à un rayonnement UV.

19. Utilisation d'une composition suivant l'une quelconque des revendications 1 à 14, dans la fabrication d'un médicament pour l'inhibition de la voie de cyclo-oxygénase.
